# EUROPEAN PATENT SPECIFICATION

(11) **EP 2 600 785 B1**
(45) Date of publication and mention of the grant of the patent: **28.01.2015**
(21) Application number: 10750033.2
(22) Date of filing: 06.08.2010
(51) Int. Cl.: A61B 18/24, A61B 17/00, A61B 18/00

(54) **LASER SAFETY DEVICE**
LASERSICHERHEITSVORRICHTUNG
DISPOSITIF DE SÉCURITÉ POUR LASER

(43) Date of publication of application: 12.06.2013
(73) Proprietor: Lascor GmbH, 81379 München (DE)
(72) Inventor: WEBER, Helmut, 81379 München (DE)
(74) Representative: Grünecker, Kinkeldey, Stockmair & Schwanhäusser
(86) International application number: PCT/EP2010/004839
(87) International publication number: WO 2012/016579

(56) References cited:
- WO-A1-2009/052866
- WO-A1-2009/072035
- WO-A2-2007/147058
- US-A1- 2010 076 299

## Description

The present invention relates to a laser safety device and a laser system comprising the laser safety device.

Catheter ablation of arrhythmogenic cardiac substrates is a technique for the treatment of various arrhythmias of the heart, based on heating or cooling particular portions of the cardiac walls in an effort to produce scars permanently blocking or preventing the electrophysiological conduction process of interest. In this respect, due to its high prevalence and stroke risk, left atrial fibrillation is of particular importance. So far, radiofrequency current has been used as the standard energy source for catheter ablation of left atrial fibrillation. However, this approach has caused severe complications due to overheating of adjacent mediastinal structures such as the esophagus, the lung, blood vessels and nerves. Thus, in an effort to avoid these complications, there is an increasing demand for alternative energy sources and safety systems able to control the energy delivery during catheter ablation of arrhythmias.

A promising alternative catheter ablation system is based on the use of infrared laser light for inducing transmural lesions of coagulation necrosis. Such a laser catheter has been published in EP1290981. This system takes advantage of the selective absorption of laser light in the various cardiac and mediastinal structures and of an improved control of the growing lesions by deflection of intracardiac potentials from the irradiation site. Nevertheless, since catheter ablation of left atrial fibrillation requires the irradiation of the rather thin atrial wall, it is desirable to have a control system that can further reduce the likelihood of thermal damage to mediastinal structures in the neighborhood of the left atrium. Furthermore, it would be helpful to have additional feedback whether laser energy is delivered by the catheter.

WO2007/147058 A2 teaches a transvascular probe.

It is an object of the present invention to address the above drawbacks of currently used catheter ablation systems.

This object is achieved with a laser safety device comprising:
- a transesophageal probe comprising a transesophageal light receiving portion for receiving laser light and a light output portion for emitting at least a part of the received light;
- an optoelectronic converting unit for producing an electric signal indicative of an amount of light emitted from the light output portion;
- an analyzing unit for processing the electric signal and for comparing the processed electric signal with a set point criterion; and
- a control unit for controlling a laser on the basis of the comparison in the analyzing unit.

The control unit is preferably configured to produce a laser control signal and/or activate a laser safety switch. In particular, the laser safety device is configured to control the laser producing the laser light to be received by the transesophageal probe. Thus, it is possible to detect laser light that has been emitted in the left atrial wall and has been transmitted into the esophagus in order to control a laser on the basis of the detected light. Thus, a feed-back loop can be established for improving the safety of a catheter ablation procedure. The light output portion is favorably arranged in the proximal section of the transesophageal probe, so that it can be coupled to an optoelectronic converter positioned outside of a patient. However, it would also be possible to integrate a photo-detector in the transesophageal probe, for instance, in the distal part of the probe to be inserted into the esophagus of a patient. The electric signal from the photo-detector could then be conducted by leads to the proximal part of the transesophageal probe and to the analyzing unit.

In a preferred embodiment, the light receiving portion has an overall diffuse reflectivity of at least 50%, in particular at least 75%. Thus, the light receiving portion can collect scattered laser light from an esophageal wall section in contact with the transesophageal probe. As a consequence, the amount of laser light transmitted through the left atrial wall can be controlled in a particular effective and reliable manner.

Preferably, the absolute maximum acceptance half-angle of the light receiving portion is at least 90°, and more particularly at least 135°. This configuration is particularly helpful in the case where the light receiving portion is placed adjacent to the left atrial wall so that light impinging laterally onto the light receiving portion can be effectively collected, guided and coupled to the optoelectronic converting unit. The maximum half-angle is preferably defined by an angle corresponding to a decrease of the sensitivity of the light receiving portion to 10% of its maximum value.

Preferably, the light receiving portion comprises a scattering body having a wall portion and a cavity surrounded by the wall portion, and wherein the volume of the cavity is greater than the volume of the wall portion. With this configuration, it is possible to improve the uniformity of the receiving characteristics of the transesophageal probe.

Favorably, the maximum diameter of the light receiving portion is 2 to 10 mm, and more preferably 4 to 8 mm. In this configuration, the transesophageal probe can be inserted through the nose of a patient, as it is routine practice for the insertion of transesophageal probes and, at the same time, the transesophageal probe can be placed in the esophagus in a stable manner. In other words, the diameter of the light receiving portion is adapted to the size of the esophagus such that the transesophageal probe is held in place by the esophageal wall, particularly in the region of the gastro-esophageal closing segment. The term "transesophageal" means that the probe is to be advanced through the esophagus and used therein.

In a preferred embodiment, the transesophageal probe further comprises an optical fiber for guiding the laser light to the light output portion, and the light receiving portion comprises a light scattering body provided at the distal end of the optical fiber, the light scattering body being hollow such that light impinging on the light scattering body at a receiving angle, with respect to the optical axis of the optical fiber, that is greater than the acceptance angle of the optical fiber can at least partially be redirected into the optical fiber. In this configuration, a standard optical fiber can be used for transmitting the received laser light to the optoelectronic converting unit. The use of a hollow light scattering body facilitates the adaption of the receiving characteristics of the light receiving portion to the acceptance angle of the optical fiber. This is achieved by multiple diffuse internal reflections in wall of the hollow light scattering body, the internal reflections eventually being directed into the cavity of the hollow scattering body.

In a further favorable embodiment, the light scattering body is formed from at least one of a spherical segment, an ellipsoid segment and a cylindrical segment, that is radially symmetric with respect to the optical axis of the optical fiber. Thus, circumferentially uniform receiving characteristics can be realized and, at the same time, a rounded distal portion that can easily be introduced into the esophagus of a patient.

In a further favorable embodiment: the light receiving portion comprises a light scattering body; the light output portion is constituted by an opening in the light scattering body; and the optoelectronic converting unit comprises a photo-detector integrated in the transesophageal probe and facing the light output portion. In such a configuration, the electrical signal can be produced at the light receiving portion and can be conducted by leads inside the transesophageal probe to an amplifier section of the optoelectronic converter and/or to the analyzing unit. Moreover, a photo-detector having a wider acceptance angle than an optical fiber can be used.

Favorably, the analyzing unit is configured to compare the processed electric signal with an upper and/or a lower threshold value and/or a characteristic signal variation with time. When the processed electrical signal is greater than an upper threshold value, it is possible to identify a situation where too much laser light is transmitted through the atrial wall into the neighboring tissue. Thus, overheating of adjacent tissue can be avoided. In the case where the processed electrical signal is smaller than a lower threshold value, a situation can be identified where the laser is switched on and light is transmitted into the atrium, but an insufficient amount of light is directed onto the atrial wall. This could indicate a situation where the laser catheter is not correctly placed on the atrial wall and laser light is partly absorbed by the blood in the atrial cavity. Using a signal variation, such as a characteristic signal curve, as the set point criterion is useful for determining whether the signal is in accordance with an expected signal variation, for instance due to a change of the optical properties of the irradiated tissue or a particular condition of the patient.

A preferred embodiment of the laser safety device further comprises a memory for storing a parameter table containing at least one upper threshold value as the set point criterion. Thus, different set point criteria can be stored for a quick retrieval during the procedure so that various treatment conditions may be identified and an appropriate control signal can be generated.

In a preferred embodiment, the transesophageal probe comprises an X-ray marker section for localizing the light receiving portion. Thus, the transesophageal probe can precisely be positioned in the vicinity of the atrial wall to be irradiated in order to allow for a reliable control of laser irradiation.

Favorably, the light receiving portion contains BaSO₄. By doing so, the scattering properties of the light receiving portion can be improved, as well as the contrast of the light receiving portion in a fluoroscopic image.

The object of the present invention is also achieved with a laser system comprising the laser safety device according to the invention and a laser, wherein the laser safety device is connected to the laser by a signal transmission path so that the control unit can decrease, via the signal transmission path, the power of laser light emitted by the laser. It is therefore possible to avoid a continuation of the laser irradiation in the case where a situation is identified that is indicative of a potential procedural complication. The signal transmission path can be realized by a cable connection or wireless. The laser irradiation could, for example, be interrupted with a control signal actuating a shutter means for preventing laser light to be coupled into the transesophageal probe. The shutter means can be a mechanical device that is brought into the beam path of the laser or an electronic device for terminating the laser emission. Anyway, the control unit can abruptly stop the laser irradiation of the heart in the case where a situation is identified that could lead to overheating of tissue.

Favorably, the signal transmission path between the laser safety device of the invention and the laser is bidirectional, so that status information whether or not the laser emits the laser light can be transmitted to the laser safety device via the signal transmission path. It is therefore possible to identify a situation where an inappropriate amount of laser light is received by the transesophageal probe while the atrial wall to be treated is irradiated with laser light. As a consequence, an inappropriate treatment situation can be identified and the laser irradiation can be interrupted.

Preferably, the laser is configured to emit infrared laser light, the laser light in particular comprising a wavelength between 850 nm and 1100 nm. Ideally, the wavelength is between 950 and 1100 nm. Laser light in the near infrared wavelength range is particularly useful for the laser-induced heating of cardiac tissue and can be easily collected and transmitted through standard optical fibers. However, other wavelengths might be used depending on the particular treatment condition.

In a preferred embodiment, the laser system further comprises a heart catheter for directing the laser light inside the heart of a patient onto endomyocardium. This configuration allows for a treatment of atrial fibrillation in the left atrium with an increased efficiency and with a reduced complication risk.

The invention will be described in more detail below with reference to preferred embodiments represented in the figures. In the drawings:
Fig. 1 shows a schematic representation of a laser safety device according to the invention;
Fig. 2 shows a schematic longitudinal section through the distal portion of a transesophageal probe according to the invention;
Fig. 3 shows a schematic longitudinal section through an alternative embodiment of a transesophageal probe according to the invention;
Fig. 4 shows a schematic representation of a laser system comprising the laser safety device according to the invention;
Fig. 5 shows a schematic diagram of a signal measured with the safety device of the invention during laser irradiation, in comparison with various setpoint criteria; and
Fig. 6 shows a schematic representation of the transesophageal probe of the invention during laser catheter ablation.

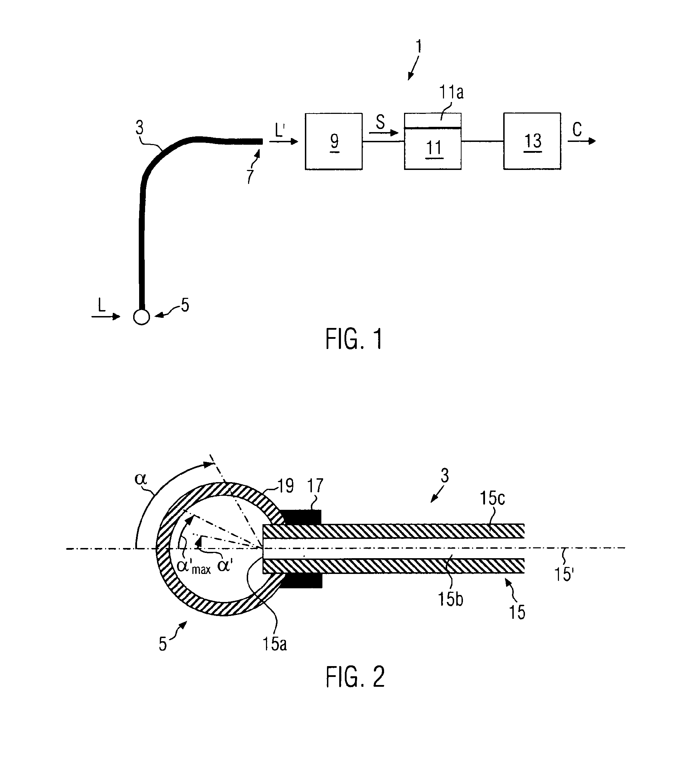

As can be seen from Fig. 1, a favorable embodiment of the laser safety device 1 according to the invention comprises a transesophageal probe 3 having a transesophageal light receiving portion 5 for receiving laser light L and a proximal light output portion 7 for directing at least a part L' of the received laser light L towards an optoelectronic converting unit 9 in order to produce an electric signal S indicative of an amount of the light L' emitted from the light output portion 7. The proximal light output portion 7 is coupled to the optoelectronic converting unit 9 by means of a connector of the SMA type or the like (not shown). The electrical signal S is processed in an analyzing unit 11, such as a computer, which is also configured to compare the processed electric signal S with a set point criterion such as a threshold value or a characteristic signal curve, which is favorably stored in a memory 11 a. The analyzing unit 11 communicates with a control unit 13 which can produce a laser control signal C or can open/close a safety switch (not shown) on the basis of the comparison in the analyzing unit 11. The control signal C is preferably adapted to control a laser, preferably a diode laser, constituting the light source of the laser light L.

Fig. 2 shows a detailed view of the distal portion of the transesophageal probe 3 comprising an optical fiber 15. The light receiving portion 5 has the shape of a hollow spherical segment attached to the optical fiber 15 close to its distal end face 15a, the core 15b of the optical fiber 15 constituting a transmission portion between the transesophageal light receiving portion 5 and the proximal light output portion 7. Fig. 2 further shows a fluoroscopic marker 17 for localizing the light receiving portion 5, which could, for example, be realized by a metallic sleeve or a coating that appears with a dark contrast in a fluoroscopic image.

As suggested by Fig. 2, the fluoroscopic marker 17 could also serve as a mounting element for fixing the transesophageal light receiving portion 5 to the optical fiber 15. However, separate mounting elements may be provided as well. It would also be possible to fix the light receiving portion 5 directly to the optical fiber 15 by means of an adhesive or a resin. The material and/or the thickness of the fiber jacket 15c of the optical fiber 15 may be specially adapted to facilitate the mounting of the transesophageal light receiving portion 5 and/or the fluoroscopic marker 17, for instance for gluing the light receiving portion 5 or crimping the fluoroscopic marker 17 onto the optical fiber 15. The cladding of the optical fiber 15 is not shown.

The light receiving portion 5 comprises a hollow light scattering body 19 which may be integrally formed with a mounting portion of the light receiving portion 5 or separately. The light scattering body 19 could be made of a solid body as well but hollow bodies are preferred for the reasons described in more detail below.

It is to be understood that the distal portion of the transesophageal probe 3, in particular the transesophageal light receiving portion 5, is to be inserted into the esophagus of a patient, whereas the proximal portion of the transesophageal probe 3 remains outside the patient. It is essential to the invention that at least a part L' of the received light L is coupled into the optoelectronic converting unit 9. Favorably, this is achieved with the setup shown in Figs. 1 and 2, wherein the light output portion 7 and the optoelectronic converting unit 9 are positioned proximally, outside the patient. In this configuration, no electrical signals need to be conducted by the transesophageal probe 3.

However, the light output portion 7 and a photo-detector constituting a part of the optoelectronic converting unit 9 could be arranged at the distal section of the transesophageal probe 3 as well. For instance, the optical fiber 15 could be replaced by catheter tubing and a photo-detector (not shown) such as a photodiode or the like could be arranged at the position of the distal end face 15a of the optical fiber 15. In this case, the light output portion 7 would be constituted by an opening in the light scattering body 19, in particular an opening surrounding and/or facing the photo-detector of the optoelectronic converting unit 9. The latter could then comprise a separate amplifying section positioned outside the patient, for adapting the electric signal S to an input of the analyzing unit 11.

Fig. 2 shows that the optical fiber 15 has a maximum acceptance angle α'ₘₐₓ formed with the optical axis 15' of the optical fiber 15, indicating that only photons that impinge on the distal end face 15a of the optical fiber 15 at an absolute receiving angle α' that is smaller than the acceptance angle α'ₘₐₓ can be guided by the optical fiber 15 to the proximal light output portion 7. Fig. 2 further shows a receiving angle α of the transesophageal light receiving portion 5, defined with respect to the optical axis 15' of the optical fiber 15 as well. In an analogous manner, a maximum acceptance angle αₘₐₓ of the light receiving portion 5 is defined such that only photons that impinge on the light receiving portion 5 at a receiving angle α that is smaller than its acceptance angle αₘₐₓ can be guided by the optical fiber 15 to the proximal light output portion 7. Depending on the shape and the materials of the light receiving portion 5, it may also be possible to define a minimum acceptance angle αₘᵢₙ of the light receiving portion 5 in the distal direction of the transesophageal probe 3. Nevertheless, it is an object of the present invention to provide a transesophageal light receiving portion 5 having a maximum acceptance angle αₘₐₓ (absolute value) that is greater than the acceptance angle α'ₘₐₓ (absolute value) of the optical fiber 15. In other words, it is an object to provide a transesophageal probe 3 that can collect light L impinging laterally onto the light receiving portion 5 such that light L that has been emitted in the left atrium and has passed through the esophageal wall can be received and transmitted to the optoelectronic converting unit 9. Thus, the absolute value of the maximum acceptance angle αₘₐₓ of the transesophageal probe 3 is favorably at least 90°, more preferably at least 135°. In case a photo-detector such as a photodiode is used instead of the distal fiber end section 15a, respective acceptance angles can be defined as well.

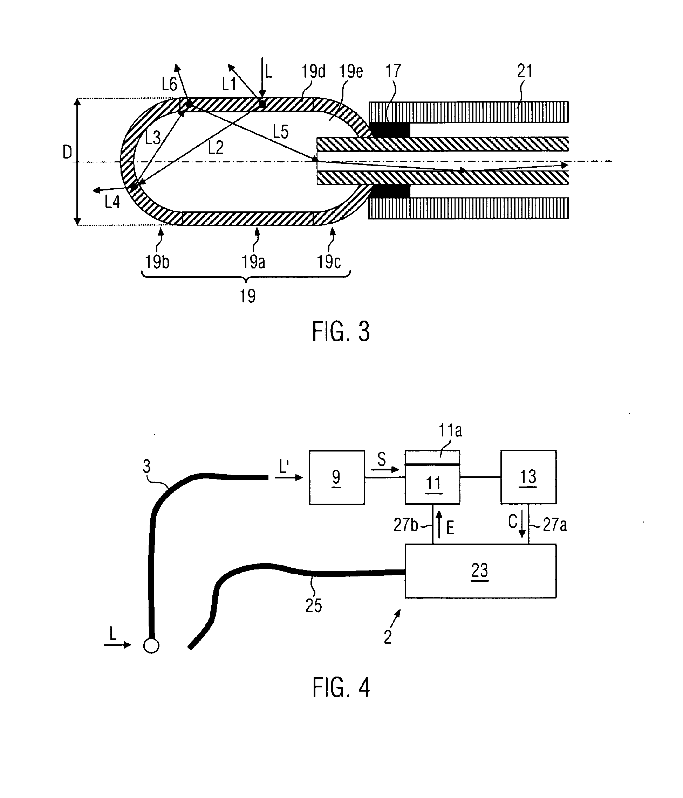

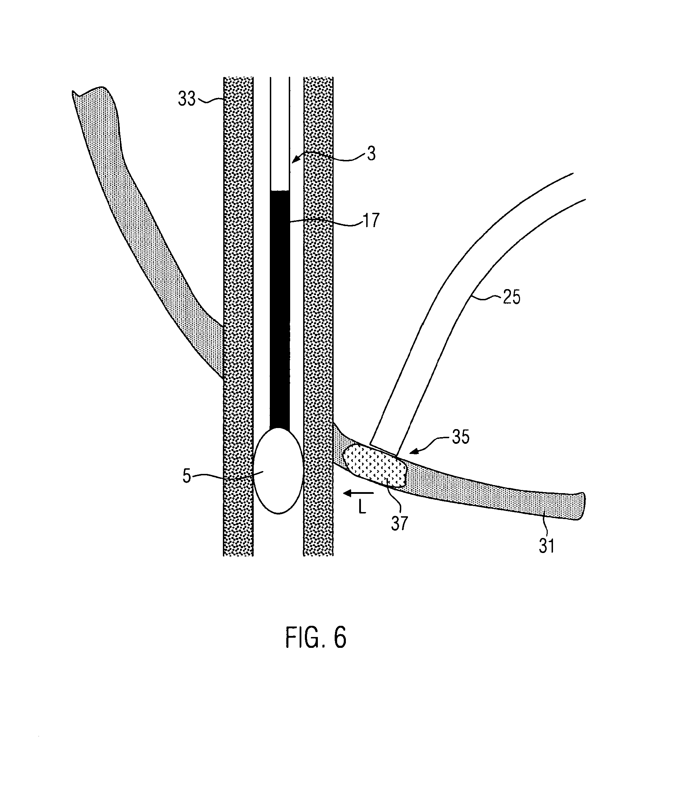

Fig. 3 shows the working principle of a transesophageal probe 3 with a wide acceptance angle αₘₐₓ. The transesophageal light receiving portion 5 comprises a hollow scattering body 19 consisting of a hollow cylindrical segment 19a and two hollow spherical segments 19b and 19c. However, as can be seen from Figs. 2 and 6, the shape of the transesophageal light receiving portion 5 is not limited to this particular shape. It would also be possible, for example, to realize the transesophageal light receiving portion 5 with a basically ellipsoid hollow scattering body 19 or a combination of differently shaped segments. It is also to be understood that the shown segments relate to different geometrical shapes and not to separate scattering bodies. Anyway, it is favorable to arrange the scattering segments 19a - 19c coaxially with respect to the optical axis 15' of the optical fiber 15 to achieve circumferentially homogeneous receiving characteristics.

Light L impinging on the light scattering body 19 is partly reflected in a diffuse manner and partly passes through the wall 19d of the light scattering body 19. In Fig. 3, this is denoted by arrows L1, L3 and L5 representing a diffuse reflection and arrows L2, L4 and L6 representing a light portion that is scattered in the wall 19d of the scattering body 19 and enters or leaves the cavity 19e thereof. As can further be seen from Fig. 3, the received light can undergo multiple internal diffuse reflections inside the scattering body 19 until a light portion L5 eventually enters the optical fiber 15. The scattering properties of the scattering body 19 are such that the number of scattering events, which are symbolized by dots in Fig. 3, before the light portion L5 enters the optical fiber 15, is sufficiently high to allow for an increase of the acceptance angle αₘₐₓ of the probe 3 such that light L laterally impinging on the light scattering body 19 can be directed into the optical fiber 15.

To this end, the light scattering body 19 is preferably made of a diffuse reflecting plastic material having a low absorption at the wavelength of the laser light L, preferably below 5%. Furthermore, scattering additives such as BaSO₄ or TiO₂ or coatings containing such materials can be used in order to increase the diffuse reflectivity of the scattering body 19. The diffuse reflectivity of the scattering body 19, defined for light L normally impinging on the wall 19d of the scattering body 19, as depicted in Fig. 3, is preferably at least 50%, ideally at least 75%. This insures that light L entering the light scattering body 19 undergoes a sufficient number of scattering events before entering the optical fiber 15. At the same time, the overall sensitivity of the light receiving portion 5 remains sufficient. In other words, by adapting the diffuse reflectivity of the scattering body 19, a trade-off between the sensitivity and the acceptance angle αₘₐₓ of the light receiving portion 5 is possible.

The maximum diameter D of the light receiving portion 5 is preferably adapted to the anatomy of a patient to be treated. Preferably, the transesophageal light receiving portion 5 is adapted for a coaxial position within the esophagus. Furthermore, the diameter D of the transesophageal light receiving portion 5 is adapted such that it can be inserted through the nose of a patient. A preferable range of the diameter D is between 2 and 10 mm. Particularly useful is a diameter D of 4 to 8 mm which allows for a smooth insertion of the transesophageal light receiving portion 5 through the nose of a patient and, at the same time, a good contact of the light receiving portion 5 with the esophageal wall.

The volume of the cavity 19e of the scattering body 19 is preferably greater than the volume of its wall 19d. This improves the smoothening of the angular receiving characteristics and decreases local differences in the receiving characteristics, for example between the segments 19a to 19c.

Fig. 3 further shows that the transesophageal probe 3 may comprise an outer probe body 21, for example a flexible plastic tube made of a biocompatible material such as polyvinylchloride, polyurethane silicone or the like, for shielding the optical fiber 15 and/or for improving the handling of the transesophageal probe 3 during insertion and positioning in the esophagus. The outer probe body 21 might also comprise a fluoroscopic marker such as a sleeve or a coating. Moreover, the mechanical properties of the proximal (outside the patient) and distal (inside the patient) portions of the outer probe body 21 might differ to further improve the insertion and positioning of the transesophageal probe 3.

Fig. 4 shows a laser system 2 according to the invention, based on the laser safety device 1 shown in Fig. 1 and further comprising a laser 23 and a laser catheter 25 for intracardiac laser irradiation. The laser 23 is preferably a diode laser emitting in the range of 850 to 1100 nm. However, other types of lasers such as a Nd:YAG laser emitting at 1064 nm could be used as well. The laser 23 is coupled to the laser safety device 1 via a bidirectional communication line 27 represented by a portion 27a for transmitting the control signal C to the laser 23, if need be, and a portion 27b for transmitting a status signal E indicative of an operation condition of the laser 23, in particular of a laser emission, to the laser safety device 1. Instead of transmitting the control signal C, it would also be possible to actuate a safety switch to turn off the laser 23. The status signal E can be transferred to either the analyzing unit 11 or the control unit 13. It is to be understood that the analyzing unit 11 and the control unit 13 can be combined in one data processing apparatus or can be implemented in the laser 23 as well. Thus, the definitions given in this respect are functional.

The laser safety device 1, in particular analyzing unit 11, preferably comprises the memory 11 a for storing a parameter table containing at least one upper (maximum) threshold value T_{U} and, preferably, also at least one lower (minimum) threshold value T_{L}, each preferably being associated with a particular laser output power of the laser 23 and the type of the transesophageal probe 3 in use. By means of the parameter table, operation ranges indicating normal and abnormal operation conditions can be defined. For instance, a normal operation range could be defined by values lying between the upper and lower thresholds T_{U}, T_{L}. Abnormal operation conditions could then be defined by values that are higher than the upper threshold T_{U} or lower than the lower threshold T_{L}, respectively.

The analyzing unit 11 is configured to process and compare the measuring signal S with set point criteria stored in the parameter table, in particular with at least one upper threshold value T_{U} and, optionally, with at least one lower threshold value T_{L}, in order to determine whether an operation or treatment condition is normal or not. The lower threshold value T_{L} is preferably used in combination with the status signal E. In case that the status signal E indicates that the laser 23 emits laser light L and the processed electrical signal S is smaller than the lower threshold T_{L}, it can be assumed that the laser catheter 25 is not correctly positioned on the cardiac wall. As a consequence, the laser emission from the laser 23 can automatically be interrupted by the control unit 13.

In the case where the processed electrical signal S is greater than the upper threshold T_{U}, it can be assumed that too much laser light L passes through the cardiac wall so that adjacent structures might be endangered. As a consequence, the laser emission from the laser 23 can automatically be interrupted by the control unit 13. In the latter case, the status signal E from the laser 23 is not necessary, but can be helpful to confirm the measurement of the optoelectronic converting unit 9.

As an alternative or supplement to the upper and lower thresholds T_{U} and T_{L}, respectively, the parameter table could also contain characteristic time-dependent variations V and/or signal curves relating to changes of the electrical signal S due to particular treatment conditions. Such a signal variation V could, for example, be induced by the ongoing coagulation of the irradiated cardiac wall or by a movement of the esophagus or the like. The analyzing unit 11 may compare the constantly updated signal S with such a characteristic signal variation V in order to identify a particular treatment condition. The control unit may then produce the control signal C on the basis of this comparison. Of course it would also be possible to combine a comparison of the processed signal S with upper and/or lower thresholds T_{U}, T_{L}, respectively, and a particular signal variation V. It would also be possible to adapt the upper and lower thresholds T_{U} and T_{L}, respectively, during the course of laser irradiation according to a known change of a treatment condition, such as a variation of optical tissue properties or the like, and/or to status information from the laser 23, for instance indicating a change of the output laser power.

The control signal C might be a signal for changing the laser output power and/or for terminating the laser emission. The control signal C could be especially adapted to the laser 23 used, for example, for setting the laser output power directly by means of the control unit 13 or for actuating a shutter means of the laser 23. It would, however, also be possible that the control signal C actuates a standard safety interlock for terminating laser irradiation.

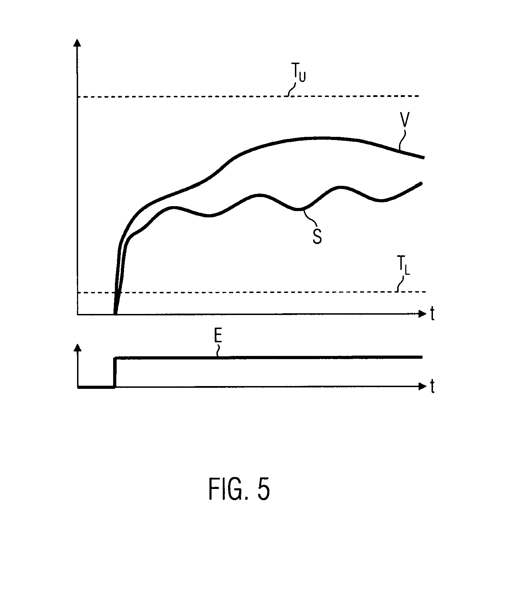

Fig. 5 shows a schematic example of the processed signal S as it might change with time t during laser irradiation, whereby the onset of laser irradiation is represented by a sudden increase of the status signal E. An example of a characteristic curve V used for comparison with the signal S is shown as well. In Fig. 5, the signal S stays below the upper threshold T_{U} and, after the onset of laser irradiation, above the lower threshold T_{L}. This would, for instance, indicate a normal treatment condition. Despite this finding, an abnormal treatment condition could, however, be identified based on a comparison of the signal S and the characteristic curve V. In this respect, it is clear that various characteristic variations or parameter ranges could be defined, for instance a range corresponding to a particular area lying between two different characteristic curves V (not shown).

The transesophageal probe 3 and the optoelectronic converting unit 9 are preferably calibrated so that the irradiance at the light receiving portion 5 or total laser power impinging on the light receiving portion 5 can be calculated. Such a calibrating function could be implemented in the analyzing unit 11, for instance in combination with a calibration light source (not shown). The optoelectronic converting unit 9 may comprise a conventional photo-detector such as a photodiode and a signal processing section such as an amplifier.

The operation of the laser safety device 1 and the laser system 2 of the invention is explained with reference to Fig. 6 which schematically shows a situation during laser irradiation of the left atrial wall 31 of a patient. Initially, the transesophageal probe 3 is inserted via the nose of the patient into the esophagus 33 of the patient. The transesophageal probe 3, in particular the position of the light receiving portion 5, is visualized by fluoroscopy, whereby the fluoroscopic marker 17, which could also extend over the whole length of the transesophageal probe 3, is used to improve the contrast of the transesophageal probe 3 in the fluoroscopic image.

As depicted in Fig. 6, the transesophageal light receiving portion 5 is placed adjacent to the left atrial wall 31 to be irradiated. The electrical signal S from the optoelectronic converting unit 9 is continuously monitored during the procedure. After a wall section 35 to be irradiated has been identified, normally by means of electrophysiological mapping or by an anatomic approach, laser light is delivered to the cardiac wall 31 by means of the laser catheter 25, whereby the status signal E indicating laser emission is transmitted to the safety device 1 via the communication line 27. In Fig. 6, the laser irradiation has already produced a coagulation lesion 37 inside the atrial wall 31. This lesion 37 is used to form an electrophysiological barrier in the atrial wall 31 at the irradiated site 35.

The analyzing unit 11 continuously compares the processed electrical signal S with at least one set point criterion stored in the parameter table. In case the electrical signal S is greater than the upper threshold T_{U,} the analyzing unit 11 communicates with the control unit 13 in order to reduce or terminate laser emission by means of the control signal C and/or a laser safety switch. Since the laser 23 continuously waits for a command or control signal C, the output power of the laser 23 is automatically reduced or the laser emission is terminated as soon as the control unit 13 sends the control signal C based on the decision in the analyzing unit 11.

In case the processed electrical signal S is smaller than the lower threshold T_{L} and the status signal E indicates laser emission, the output power of the laser 23 is increased or the laser emission is terminated by sending a corresponding control signal C in the same manner as described above. The decision whether it is preferable to increase the laser power or to terminate the laser emission may depend on the particular treatment condition and the processed electrical signal S.

With the laser safety device 1 and the laser system 2 according to the invention it is possible to reduce the risk of overheating anatomical structures of the mediastinum during laser irradiation of a cardiac wall, particularly of the left atrial wall. In combination with status information E from the laser 23, and depending on the particular treatment situation, it is possible to shorten an ineffective laser irradiation due to an inappropriate laser catheter position.

The transesophageal probe 3 of the invention can easily be inserted through the nose of a patient so that the control function of the laser safety device 1 of the invention can easily be implemented in a catheter laboratory. Particularly for the treatment of left atrial fibrillation, which requires numerous coagulation lesions to be produced in the thin atrial wall, it is possible to reduce the risk of damaging adjacent tissue.

The described embodiments and variants can be combined with each other in a technically favorable manner, as long as laser light can be received in the esophagus of a patient and an electric signal based on the transesophageal light measurement can be compared with a set point criterion in order to control a therapeutic light source.

## Claims

1. A laser safety device (1) comprising:
- a transesophageal probe (3) comprising a transesophageal light receiving portion (5) for receiving laser light (L) and a light output portion (7) for emitting at least a part (L') of the received light;
- an optoelectronic converting unit (9) for producing an electric signal (S) indicative of an amount of the light (L') emitted from the light output portion;
- an analyzing unit (11) for processing the electric signal (S) and for comparing the processed electric signal with a set point criterion; and
- a control unit (13) for controlling a laser on the basis of the comparison in the analyzing unit.

2. The laser safety device according to claim 1, wherein the light receiving portion (5) has an overall diffuse reflectivity of at least 50%, and more particularly at least 75%.

3. The laser safety device according to claim 1 or 2, wherein the absolute maximum acceptance half angle (αₘₐₓ) of the light receiving portion (5) is at least 90°, and more particularly at least 135°.

4. The laser safety device according to at least one of the preceding claims, wherein the light receiving portion (5) comprises a scattering body (19) having a wall portion (19d) and a cavity (19e) surrounded by the wall portion, and wherein the volume of the cavity is greater than the volume of the wall portion.

5. The laser safety device according to at least one of the preceding claims, wherein the maximum diameter (D) of the light receiving portion (5) is 2 to 10 mm, and in particular 4 to 8 mm.

6. The laser safety device according to at least one of the preceding claims, wherein the transesophageal probe (3) further comprises an optical fiber (15) for guiding the laser light to the light output portion (7), and wherein the light receiving portion (5) comprises a light scattering body (19) provided at the distal end portion (15a) of the optical fiber, the light scattering body being hollow such that light (L) impinging on the light scattering body at a receiving angle (α) with respect to the optical axis (15') of the optical fiber, that is greater than the acceptance angle (αₘₐₓ) of the optical fiber, is at least partially redirectable into the optical fiber.

7. The laser safety device according to claim 6, wherein the light scattering body (19) is formed by at least one of a spherical segment, an ellipsoid segment and a cylindrical segment (19a, 19b, 19c), that is radially symmetric with respect to the distal end portion (15a) of the optical fiber (15).

8. The laser safety device according to at least one of claims 1 to 5, wherein: the light receiving portion (5) comprises a light scattering body (19); the light output portion (7) is constituted by an opening in the light scattering body; and the optoelectronic converting unit (9) comprises a photo- detector integrated in the trans-esophageal probe (3) and facing the light output portion.

9. The laser safety device according to at least one of the preceding claims, wherein the analyzing unit (11) is adapted to compare the processed electric signal with an upper and/or a lower threshold value (Tᵤ, T_{L}) and/or a characteristic signal variation (V) with time, as the set point criterion.

10. The laser safety device according to at least one of the preceding claims, wherein the transesophageal probe (3) comprises an X-ray marker section (17) for localizing the light receiving portion (5).

11. The laser safety device according to at least one of the preceding claims, wherein the light receiving portion (5) contains BaSO₄.

12. A laser system (2) comprising the laser safety device according to at least one of the preceding claims and a laser (23), wherein the laser safety device (1) is connected to the laser (23) by a signal transmission path (27a) so that the power of laser light emitted by the laser is decreasable by the control unit (13) via the signal transmission path.

13. The laser system according to claim 12, wherein the signal transmission path (27a, 27b) between the laser safety device (1) and the laser (23) is bidirectional, so that status information (E) whether or not the laser emits the laser light is transmittable to the laser safety device via the signal transmission path.

14. The laser system according to claim 12 or 13, wherein the laser (23) is configured to emit infrared laser light, the laser light in particular comprising a wavelength between 850 nm and 1 100 nm.

15. The laser system according to any of claims 12 to 14, further comprising a heart catheter (25) for directing the laser light inside of the heart onto endocardium.

## Patentansprüche

1. Eine Lasersicherheitsvorrichtung (1) umfassend:
- eine transösophageale Sonde (3) umfassend einen transösophagealen Lichtempfangsabschnitt (5) zum Empfangen von Laserlicht (L) und einen Lichtabgabeabschnitt (7) zum Aussenden von wenigstens einem Anteil (L') des empfangenen Lichts;
- eine optoelektronische Umwandlungseinheit (9) zum Erzeugen eines elektrischen Signals (S), das eine Menge des von dem Lichtabgabeabschnitt ausgesandten Lichts angibt;
- eine Analyseeinheit (11) zum Verarbeiten des elektrischen Signals (S) und zum Vergleichen des verarbeiteten elektrischen Signals mit einem Vorgabekriterium; und
- eine Steuereinheit (13) zum Steuern eines Lasers auf der Basis des Vergleichs in der Analyseeinheit.

2. Die Lasersicherheitsvorrichtung nach Anspruch 1, wobei der Lichtempfangsabschnitt (5) eine diffuse Gesamtreflektivität von wenigstens 50% aufweist, und insbesondere von wenigstens 75%.

3. Die Lasersicherheitsvorrichtung nach Anspruch 1 oder 2, wobei der absolute maximale Akzeptanzhalbwinkel (αₘₐₓ) des Lichtempfangsabschnitts (5) wenigstens 90° beträgt, und insbesondere wenigstens 135°.

4. Die Lasersicherheitsvorrichtung nach wenigstens einem der vorigen Ansprüche, wobei der Lichtempfangsabschnitt (5) einen Streukörper (19) umfasst, der einen Wandabschnitt (19d) und einen von dem Wandabschnitt umgebenen Hohlraum (19e) aufweist, und wobei das Volumen des Hohlraums größer ist als das Volumen des Wandabschnitts.

5. Die Lasersicherheitsvorrichtung nach wenigstens einem der vorigen Ansprüche, wobei der maximale Durchmesser (D) des Lichtempfangsabschnitts (5) 2 bis 10 mm beträgt, und insbesondere 4 bis 8 mm.

6. Die Lasersicherheitsvorrichtung nach wenigstens einem der vorigen Ansprüche, wobei die transösophageale Sonde (3) ferner eine optische Faser (15) zum Leiten des Laserlichts zum Lichtabgabeabschnitt (7) umfasst, und wobei der Lichtempfangsabschnitt (5) einen Lichtstreukörper (19) umfasst, der am distalen Endabschnitt (15a) der optischen Faser ausgebildet ist, wobei der Lichtstreukörper hohl ist, derart dass Licht (L), das auf den Lichtstreukörper mit einem Empfangswinkel (α) bezüglich der optischen Achse (15') der optischen Faser auftrifft, der größer ist als der Akzeptanzwinkel (αₘₐₓ) der optischen Faser, zumindest anteilig in die optische Faser zurückleitbar ist.

7. Die Lasersicherheitsvorrichtung nach Anspruch 6, wobei der Lichtstreukörper (19) wenigstens von einem sphärischen Segment, einem ellipsoiden Segment und/oder einem zylindrischen Segment (19a, 19b, 19c) gebildet wird, das radialsymmetrisch bezüglich des distales Endabschnitts (15a) der optischen Faser (15) ausgebildet ist.

8. Die Lasersicherheitsvorrichtung nach wenigstens einem der Ansprüche 1 bis 5, wobei: der Lichtempfangsabschnitt (5) einen Lichtstreukörper (19) umfasst; der Lichtabgabeabschnitt (7) durch eine Öffnung im Lichtstreukörper ausgebildet ist; und die optoelektronische Umwandlungseinheit (9) einen Fotodetektor umfasst, der in die transösophageale Sonde (3) integriert ist und dem Lichtabgabeabschnitt zugewandt ist.

9. Die Lasersicherheitsvorrichtung nach wenigstens einem der vorigen Ansprüche, wobei die Analyseeinheit (11) ausgebildet ist, das verarbeitete elektrische Signal mit einem oberen und/ oder einem unteren Schwellenwert (T_{U}, T_{L}) und/oder einer charakteristischen zeitlichen Signalschwankung (V) als Vorgabekriterium zu vergleichen.

10. Die Lasersicherheitsvorrichtung nach wenigstens einem der vorigen Ansprüche, wobei die transösophageale Sonde (3) einen Röntgenkontrastabschnitt (17) zum Lokalisieren des Lichtempfangsabschnitts (5) umfasst.

11. Die Lasersicherheitsvorrichtung nach wenigstens einem der vorigen Ansprüche, wobei der Lichtempfangsabschnitt (5) BaSO₄ umfasst.

12. Ein Lasersystem (2) umfassend die Lasersicherheitsvorrichtung nach wenigstens einem der vorigen Ansprüche und einen Laser (23), wobei die Lasersicherheitseinrichtung (1) mit dem Laser (23) durch einen Signalübertragungsweg (27a) derart verbunden ist, dass die Leistung des von dem Laser ausgesandten Laserlichts durch die Steuereinheit (13) über den Signalübertragungsweg reduzierbar ist.

13. Das Lasersystem nach Anspruch 12, wobei der Signalübertragungsweg (27a, 27b) zwischen der Lasersicherheitsvorrichtung (1) und dem Laser (23) bidirektional ist, sodass Statusinformation (E), dahingehend ob der Laser Laserlicht aussendet oder nicht, zur Lasersicherheitsvorrichtung über den Signalübertragungsweg übertragbar ist.

14. Das Lasersystem nach Anspruch 12 oder 13, wobei der Laser (23) zum Aussenden von infrarotem Laserlicht ausgebildet ist, wobei das Laserlicht insbesondere eine Wellenlänge zwischen 850 nm und 1100 nm umfasst.

15. Das Lasersystem nach einem der Ansprüche 12 bis 14, ferner mit einem Herzkatheter (25) zum intrakardialen Richten des Laserlichts auf Endokard.

## Revendications

1. Dispositif de sécurité pour laser (1) comprenant :
une sonde transoesophagienne (3) comprenant une partie de réception de lumière transoesophagienne (5) pour recevoir une lumière laser (L) et une partie de sortie de lumière (7) pour émettre au moins une partie (L') de la lumière reçue ;
une unité de conversion optoélectronique (9) pour produire un signal électrique (S) indiquant la quantité de lumière (L') émise par la partie de sortie de lumière ;
une unité d'analyse (11) pour traiter le signal électrique (S) et pour comparer le signal électrique traité à un critère de point de consigne ; et
une unité de commande (13) pour commander un laser en se basant sur la comparaison effectuée dans l'unité d'analyse.

2. Dispositif de sécurité pour laser selon la revendication 1, dans lequel la partie de réception de lumière (5) possède une réflexion diffuse globale d'au moins 50 %, et plus particulièrement, d'au moins 75 %.

3. Dispositif de sécurité pour laser selon la revendication 1 ou 2, dans lequel le demi-angle d'acceptation maximum absolu (σₘₐₓ) de la partie de réception de lumière (5) est d'au moins 90°, et plus particulièrement, d'au moins 135°.

4. Dispositif de sécurité pour laser selon au moins une des revendications précédentes, dans lequel la partie de réception de lumière (5) comprend un corps de dispersion (19) comportant une partie de paroi (19d) et une cavité (19e) entourée par la partie de paroi, et dans lequel le volume de la cavité est plus grand que le volume de la partie de paroi.

5. Dispositif de sécurité pour laser selon au moins une des revendications précédentes, dans lequel le diamètre maximum (D) de la partie de réception de lumière (5) est de 2 mm à 10 mm, et en particulier de 4 mm à 8 mm.

6. Dispositif de sécurité pour laser selon au moins une des revendications précédentes, dans lequel la sonde transoesophagienne (3) comprend en outre une fibre optique (15) pour guider la lumière laser vers la partie de sortie de lumière (7), et dans lequel la partie de réception de lumière (5) comprend un corps de dispersion de lumière (19) prévu au niveau de la partie d'extrémité distale (15a) de la fibre optique, le corps de dispersion de lumière étant creux, de sorte que la lumière (L) frappant le corps de dispersion de lumière avec un angle de réception (α) par rapport à l'axe optique (15') de la fibre optique, qui est plus grand que l'angle d'acceptation (αₘₐₓ) de la fibre optique, peut au moins être partiellement redirigée dans la fibre optique.

7. Dispositif de sécurité pour laser selon la revendication 6, dans lequel le corps de dispersion de lumière (19) est formé par au moins un segment parmi un segment sphérique, un segment ellipsoïde et un segment cylindrique (19a, 19b, 19c), qui est radialement symétrique par rapport à la partie d'extrémité distale (15a) de la fibre optique (15).

8. Dispositif de sécurité pour laser selon au moins une des revendications 1 à 5, dans lequel : la partie de réception de lumière (5) comprend un corps de dispersion de lumière (19) ; la partie de sortie de lumière (7) est constituée d'une ouverture dans le corps de dispersion de lumière ; et l'unité de conversion optoélectronique (9) comprend un photodétecteur intégré dans la sonde transoesophagienne (3) et tourné vers la partie de sortie de lumière.

9. Dispositif de sécurité pour laser selon au moins une des revendications précédentes, dans lequel l'unité d'analyse (11) est adaptée à comparer le signal électrique traité à une valeur de seuil supérieure et/ou à une valeur de seuil inférieure (T_{U}, T_{L}) et/ou à une variation de signal caractéristique (V) avec le temps, en tant que critère de point de consigne.

10. Dispositif de sécurité pour laser selon au moins une des revendications précédentes, dans lequel la sonde transoesophagienne (3) comprend une section de repère radiographique (17) pour localiser la partie de réception de lumière (5).

11. Dispositif de sécurité pour laser selon au moins une des revendications précédentes, dans lequel la partie de réception de lumière (5) contient du BaSO₄.

12. Système à laser (2) comprenant le dispositif de sécurité pour laser selon au moins l'une des revendications précédentes et un laser (23), dans lequel le dispositif de sécurité pour laser (1) est relié au laser (23) par un trajet de transmission de signal (27a) de sorte que l'unité de commande (13) peut faire diminuer la puissance de la lumière laser émise par le laser par l'intermédiaire du trajet de transmission de signal.

13. Système à laser selon la revendication 12, dans lequel le trajet de transmission de signal (27a, 27b) entre le dispositif de sécurité pour laser (1) et le laser (23) est bidirectionnel, de sorte que des informations d'état (E) relatives à l'émission ou à l'absence d'émission de la lumière laser par le laser peuvent être transmises au dispositif de sécurité pour laser par l'intermédiaire du trajet de transmission de signal.

14. Système à laser selon l'une quelconque des revendications 12 ou 13, dans lequel le laser (23) est configuré pour émettre une lumière laser infrarouge, la lumière laser comprenant en particulier une longueur d'onde comprise entre 850 nm et 1100 nm.

15. Système à laser selon l'une quelconque des revendications 12 à 14, comprenant en outre un cathéter cardiaque (25) pour diriger la lumière laser du coeur sur l'endocarde.
